# EUROPEAN PATENT APPLICATION

(11) **EP 4 393 418 A1**
(43) Date of publication of application: **03.07.2024**
(21) Application number: 21954777.5
(22) Date of filing: 02.11.2021
(51) Int. Cl.: A61B 17/221

(54) **ADJUSTABLE THROMBECTOMY APPARATUS**

(30) Priority: 24.08.2021 CN 202110976158
(71) Applicant: Beijing Taijieweiye Technology Co., Ltd., Beijing 101204 (CN)
(72) Inventor: MU, Lei, Beijing 101204 (CN); CHENG, Wenjie, Beijing 101204 (CN); XU, Yongsong, Beijing 101204 (CN); WANG, Mengjing, Beijing 101204 (CN); FAN, Wenji, Beijing 101204 (CN); WU, Yongzhao, Beijing 101204 (CN)
(74) Representative: Glück Kritzenberger Patentanwälte PartGmbB
(86) International application number: PCT/CN2021/128043
(87) International publication number: WO 2023/024257

(57) **Abstract**

An adjustable thrombectomy device, comprising: an adjustable handle (1) having a hollow structure, the adjustable handle (1) comprising an adjustment knob (2); an adjusting filament (6), a proximal end thereof being connected to the adjustment knob (2), so that rotation and/or axial movement can be achieved by adjusting the adjustment knob (2); a conveying conduit (3), a proximal end thereof being inserted into the hollow structure of the adjustment knob (2) and sleeved outside the adjusting filament (6); a thrombectomy stent (4), a proximal end thereof being connected to the distal end of the adjusting filament (6), and expansion or contraction being achieved under pulling force of the adjusting filament (6), it also being possible, by means of the adjustment knob (2) and the adjusting filament (6), to adjust an opening sequence of units of the thrombectomy stent (4); and a guide component (5) connected to the distal end of the thrombectomy stent (4). When in use, expansion and opening of the thrombectomy stent (4) at a pathological site are achieved by means of rotating or pushing the adjustment knob (2), thereby capturing a thrombus and removing the thrombus along with the device.

## Description

The application claims the priority to Chinese Patent Application No. 202110976158.2, entitled "Adjustable Thrombectomy Device", filed with the Patent Office of the People's Republic of China on August 24, 2021.

### BACKGROUND OF THE INVENTION

### 1. Technical Field

The present disclosure relates to the field of medical instruments, and particularly relates to an adjustable thrombectomy device.

### 2. Description of Related Art

Acute ischemic stroke is a nerve tissue injury induced by avascular necrosis of regional brain tissue due to sudden blockage of cerebral blood flow, which is the principal disease causing death and disability of the middle aged and elderly people. With the increase of aged population and improvement of living standard, the incidence rate of cerebrovascular disease increases. Revascularization is the key to treating acute ischemic stroke. Conventional methods for treating acute ischemic stroke at present include two categories: endovascular thrombolysis and mechanical thrombectomy.

Endovascular thrombolysis means that a thrombolytic agent is injected near a focus in a blood vessel of a lesion through a conduit to form a very high concentration of thrombolytic agent instantaneously in the regional focus so as to accelerate the thrombolysis speed, thereby increasing the chance of revascularization. First of all, intravenous thrombolysis shall be conducted within 3 hours of onset, and the time window for intra-arterial thrombolysis is within 6 hours, so that a very few patients can receive thrombolytic therapy; secondly, thrombolytic therapy is only suitable for thrombi with small sizes, and has an unsatisfactory effect on thrombi with large sizes; a great dosage of thrombolytic drug is needed to dissolve thrombi, which, however, easily induces various complications, so that the risk is high. To solve the problems in intravascular thrombolysis, embolizing thrombi are removed by means of mechanical thrombectomy for patients when drug thrombolysis is not suitable.

At present, a common laser engraving integral thrombectomy stent with defects such as poor ability to pass distal lesion, thrombus escape and high complications as the thrombus is pulled to turn to easily damage the blood vessel. Although the laser engraving thrombectomy stent composed of a plurality of units can improve the lesion passing ability and the thrombus grabbing effect, the damage of the blood vessel by the thrombectomy process is not reduced due to high strength of the laser engraving stent.

### BRIEF SUMMARY OF THE INVENTION

To overcome defects in the prior art, the object of the present disclosure is to provide an adjustable thrombectomy device to at least partially solve the above technical problems.

In order to achieve the above object, the present disclosure provides an adjustable thrombectomy device, including:
an adjustable handle having a hollow structure and including an adjustment knob;
an adjusting filament having a proximal end connected to the adjustment knob, wherein the adjusting filament is configured to be adjusted by the adjustment knob to rotate and/or move axially;
a conveying conduit having a proximal end inserted into the hollow structure of the adjustable handle, wherein the conveying conduit is sleeved outside the adjusting filament; and
a thrombectomy stent having a proximal end connected to a distal end of the adjusting filament, wherein the thrombectomy stent is configured to be pulled by the adjusting filament to expand or contract.

In some solutions, the adjustment knob has at least one of the following adjusting modes:
a rotating mode and a push-pull mode.
In some embodiments, the device further includes:
a guide component connected to the distal end of the thrombectomy stent, wherein the guide component includes a developing spring, and a ball head is arranged on the developing spring.

In some solutions, the thrombectomy stent includes one mesh basket or a plurality of mesh baskets connected in series.

In some solutions, developable labels are arranged at proximal ends and/or distal ends of at least part of the mesh baskets.

In some solutions, the mesh basket is provided with a large mesh capturing region or a thrombus capturing depressed region.

In some solutions, the mesh basket is of a metal wire woven structure or a laser engraving structure.

In some solutions, a wire diameter range of metal wires of the metal wire woven structure is 0.03-0.12 mm.

In some solutions, a width range of stent struts of the laser engraving structure is 0.04-0.12 mm, and the thickness range of the stent struts is 0.03-0.10 mm.

In some solutions, a wire diameter range of the developing spring is 0.02-0.06 mm;
a length range of the developing spring is 5-15 mm; and
a diameter range of the developing spring is 0.2-0.5 mm.

In some solutions, an adjustable range of an overall length of the thrombectomy stent is 10-60 mm; and
the diameter adjustable range of the thrombectomy stent is 1.0-6.0 mm.

In some solutions, the mesh basket at the distal end of the thrombectomy stent is of a disc-like structure in an open state, and an outer diameter of an disc-like structure is adaptive to the inner diameter of the blood vessel.

In some solutions, the spatial form of the mesh basket of the thrombectomy stent in the open state is any one or more combinations o f ellipsoidal shape, spindle shape, rugby shape, bulb shape, gourd shape, pear shape and dumb-bell shape.

The embodiments of the present disclosure provide an adjustable thrombectomy device. The adjustable handle is at the proximal end of the device, the distal end of the adjustable handle is connected to the conveying conduit, the adjusting filament is arranged in the conveying conduit, the proximal end of the adjusting filament is connected to the adjustment knob on the adjustable handle, and the distal end of the adjusting filament is connected to the thrombectomy stent. The adjusting filament is pulled by rotating or horizontally pushing the adjustment knob, so that the thrombectomy stent is expanded and opened under the action of the adjusting filament, so as to capture the thrombus. The thrombectomy stent can cling to the inner wall of the blood vessel to prevent thrombus from escaping. Meanwhile, the guide component at the distal end of the thrombectomy stent uses the developable spring with the ball head, so that the distal passing ability thereof is better.

### BRIEF DESCRIPTION OF THE SEVERAL VIEWS OF THE DRAWINGS

FIG. 1 is a schematic structural diagram of an adjustable thrombectomy device according to a first embodiment of the present disclosure;
FIG. 2 is an amplified schematic diagram of a distal structure of the adjustable thrombectomy device according to a first embodiment of the present disclosure;
FIG. 3 is a first working schematic diagram of the adjustable thrombectomy device according to a first embodiment of the present disclosure;
FIG. 4 is a second working schematic diagram of the adjustable thrombectomy device according to a first embodiment of the present disclosure;
FIG. 5a is a schematic diagram of an initial state of a thrombectomy stent at a lesion part according to a first embodiment of the present disclosure;
FIG. 5b is a schematic diagram of a half-open state of the thrombectomy stent at a lesion part according to a first embodiment of the present disclosure;
FIG. 5c is a schematic diagram of a completely open state of the thrombectomy stent at a lesion part according to a first embodiment of the present disclosure;
FIG. 6a is a schematic diagram of an open state of a thrombectomy stent of an adjustable thrombectomy device at a lesion part according to a second embodiment of the present disclosure;
FIG. 6b is a schematic structural diagram of a mesh basket of the adjustable thrombectomy device according to a second embodiment of the present disclosure;
FIG. 7 is a schematic diagram of an open state of a thrombectomy stent of an adjustable thrombectomy device at a lesion part according to a third embodiment of the present disclosure;
FIG. 8 is a schematic diagram of an open state of the thrombectomy stent of an adjustable thrombectomy device at a lesion part according to a fourth embodiment of the present disclosure;
FIG. 9 is a schematic diagram of an open state of a thrombectomy stent of an adjustable thrombectomy device at a lesion part according to a fifth embodiment of the present disclosure;
FIG. 10 is a schematic diagram of an open state of a thrombectomy stent of an adjustable thrombectomy device at a lesion part according to a sixth embodiment of the present disclosure;
FIG. 11 is a schematic diagram of an open state of a thrombectomy stent of an adjustable thrombectomy device at a lesion part according to a seventh embodiment of the present disclosure;
FIG. 12 is a schematic diagram of an open state of a thrombectomy stent of an adjustable thrombectomy device at a lesion part according to an eighth embodiment of the present disclosure;
FIG. 13 is a schematic diagram of an open state of a thrombectomy stent of an adjustable thrombectomy device at a lesion part according to a ninth embodiment of the present disclosure;
FIG. 14 is a schematic diagram of an open state of a thrombectomy stent of an adjustable thrombectomy device at a lesion part according to a tenth embodiment of the present disclosure;
FIG. 15 is a schematic diagram of an open state of a thrombectomy stent of an adjustable thrombectomy device at a lesion part according to an eleventh embodiment of the present disclosure;
FIG. 16 is a schematic diagram of an open state of a thrombectomy stent of an adjustable thrombectomy device at a lesion part according to a twelfth embodiment of the present disclosure;
FIG. 17 is a schematic diagram of an open state of a thrombectomy stent of an adjustable thrombectomy device at a lesion part according to a thirteenth embodiment of the present disclosure;
FIG. 18 is a schematic diagram of an open state of a thrombectomy stent of an adjustable thrombectomy device at a lesion part according to a fourteenth embodiment of the present disclosure;
FIG. 19 is a schematic diagram of an open state of a thrombectomy stent of an adjustable thrombectomy device at a lesion part according to a fifteenth embodiment of the present disclosure.

### DETAILED DESCRIPTION OF THE INVENTION

The technical solution of the present disclosure will be further described in detail below in combination with drawings and embodiments.

The embodiments of the present disclosure provide an adjustable thrombectomy device. An adjustment knob of an adjustable handle is rotated and/or push-pulled to pull an adjusting filament in a conveying conduit, and a thrombectomy stent is pulled by the adjusting filament to expand or contract to capture the thrombus. Meanwhile, a distal end of the thrombectomy stent in the open state can cling to the inner wall of the blood vessel, so as to prevent the thrombus from escaping. A guide component at the distal end of the thrombectomy stent uses a developable spring with a ball head, so that the distal passing ability thereof is better.

As discussed herein, the term "distal end" or "proximal end" are used for describing the position or direction relative to a handheld end of a treating physician or a medical interventional physician hereafter. The "distal end" or "distal side" is the position away from the direction of the handheld end of the physician or the interventional physician. The "proximal end" or "near end" is the position close to the direction of the handheld end of the physician or the interventional physician. The terms "blockage", "thrombus: or "occlusion" can be used interchangeably.

The present disclosure is described in detail below through specific embodiments. It shall be understood that the embodiments below are not intended to limit the present disclosure. Those skilled in the art are capable of thinking of arranging and combining specific features in the embodiments to form other similar solutions based on the concept of the present disclosure.

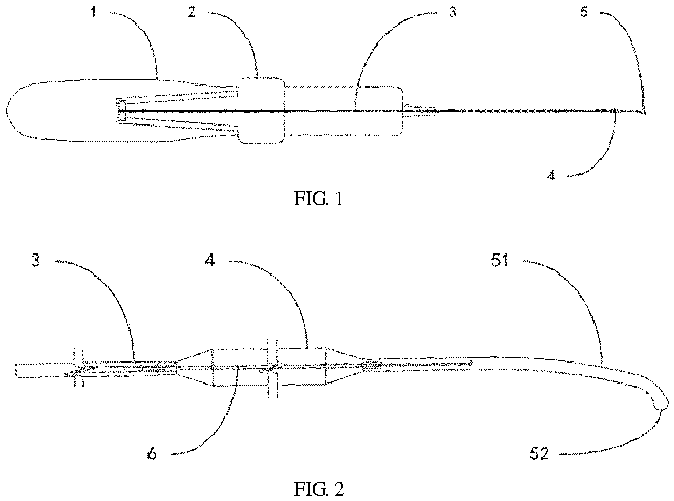

FIG. 1 is a schematic structural diagram of an adjustable thrombectomy device according to a first embodiment of the present disclosure; and FIG. 2 is an amplified schematic diagram of a distal structure of the adjustable thrombectomy device according to a first embodiment of the present disclosure. As shown in FIG. 1 and FIG. 2, the adjustable thrombectomy device includes an adjustable handle 1, an adjustment knob 2, a conveying conduit 3, a thrombectomy stent 4, a guide component 5 and an adjusting filament 6. The guide component 5 includes a developing spring 51 and a ball head 52.

The adjustable handle 1 is at a proximal end of the adjustable thrombectomy device and is held by a physician to operate. The adjustable handle 1 has a hollow structure and includes the adjustment knob 2.

A proximal end of the adjusting filament 6 is connected to the adjustment knob 2, and the adjusting filament 6 can be adjusted by the adjustment knob 2 to rotate and/or move axially, i.e., the adjusting filament 6 is tensioned and relaxed by operating the adjustment knob 2. The operating modes of the adjustment knob 2 can be set as a rotating mode and a push-pull mode.

A proximal end of the conveying conduit 3 is inserted into the hollow structure of the adjustable handle 1 and the conveying conduit 3 is sleeved outside the adjusting filament 6.

A proximal end of the thrombectomy stent 4 is connected to a distal end of the adjusting filament 6, the thrombectomy stent 4 is a shape-adjustable thrombectomy device, and the adjusting filament 6 can be pulled by operating the adjustment knob 2, so that the diameter of the thrombectomy stent 4, under the stretching action of the adjusting filament 6, is adjusted from minor to major, and the length is shortened to a certain extent in the axial direction.

The guide component 5 is further connected to the distal end of the thrombectomy stent 4, wherein the guide component 5 includes the developing spring 51, and the ball head 52 is arranged on the developing spring 51.The developing spring 51 is of a flexible spring structure, and the ball head 52 is of a ball cap structure, so that the passing ability of the distal end of the thrombectomy device in the blood vessel can be remarkably improved.

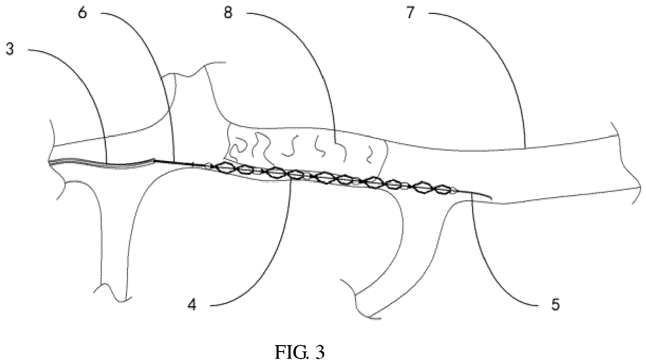

FIG. 3 is a first working schematic diagram of the adjustable thrombectomy device according to a first embodiment of the present disclosure. As shown in FIG. 3, under the action of the conveying conduit 3 and the adjusting filament 6, the thrombectomy stent 4 arrives at the thrombus 8 at a lesion part located in the blood vessel 7.

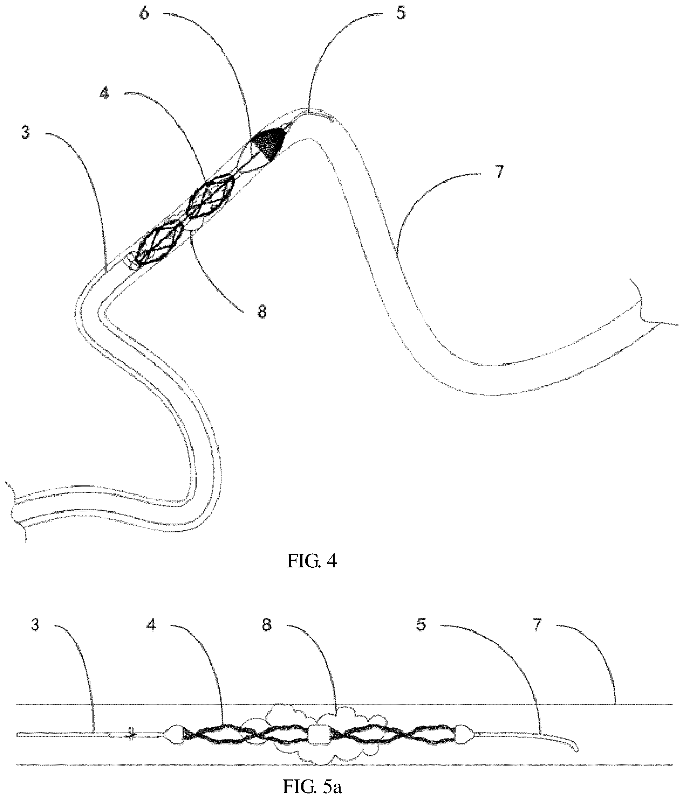

FIG. 4 is a second working schematic diagram of the adjustable thrombectomy device according to a first embodiment of the present disclosure. As shown in FIG. 4, the thrombectomy stent 4 in a completely open state captures the thrombus 8 within in the blood vessel 7.

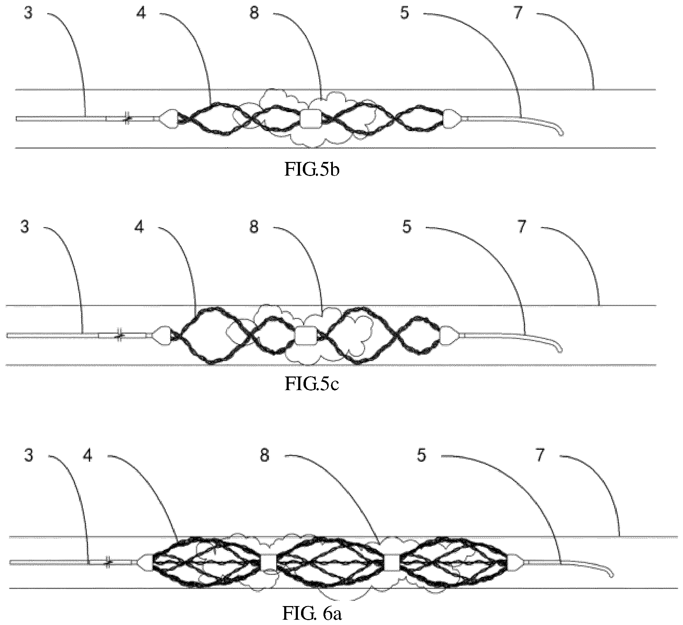

FIG. 5a to FIG. 5c are schematic diagrams showing changes of the working state of the thrombectomy stent of the adjustable thrombectomy device according to a first embodiment of the present disclosure, wherein FIG. 5a is the schematic diagram of the initial state of the thrombectomy stent 4 just arriving at the lesion part, and at this time, the thrombectomy stent 4 is not radially regulated; at the moment, the thrombectomy stent 4 is regulated by operating the adjustment knob 2, the thrombectomy stent 4 is slowly turned into the median tensioned state shown in FIG. 5b, and at this time, the thrombectomy stent 4 is in a semi-expanded state: the thrombectomy stent 4 is tensioned and regulated by the adjustment knob 2, so that the thrombectomy stent 4 is further radially dilated to cling to the inner wall of the blood vessel 7, so as to capture the thrombus 8 through the depressed region in the middle of the thrombectomy stent 4.

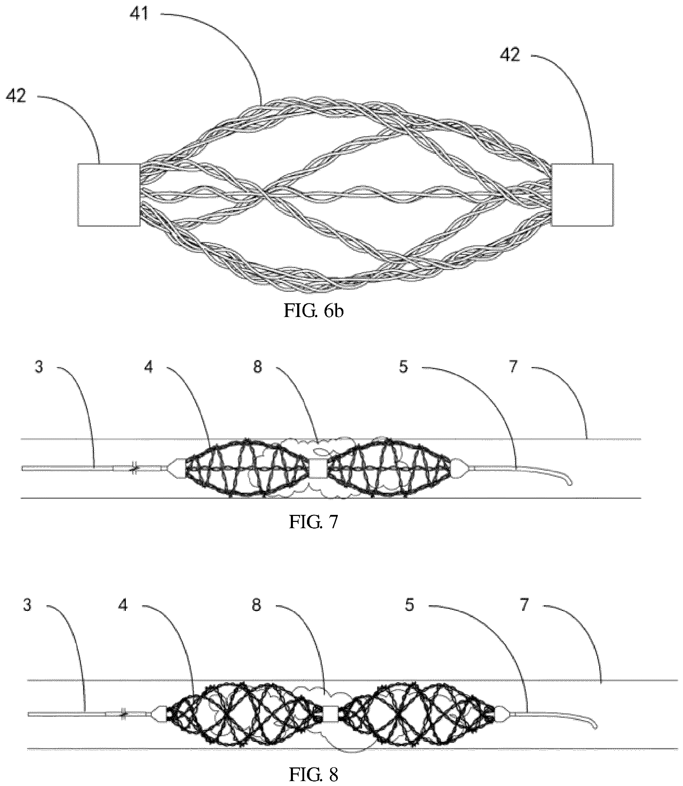

FIG. 6a is a schematic diagram of an open state of a thrombectomy stent of an adjustable thrombectomy device at a lesion part according to a second embodiment of the present disclosure; and FIG. 6b is a schematic structural diagram of a mesh basket of the adjustable thrombectomy device according to a second embodiment of the present disclosure. As shown in FIG. 6a and FIG. 6b, the thrombectomy stent 4 may include one mesh basket or a plurality of mesh baskets 41 connected in series. Developable labels 42 are arranged at both ends of the mesh basket 41. When the thrombectomy stent in the multi-serial structure is opened, there may exist various open space states for different unit stent structures. The mesh basket 41 may be provided with a large mesh capturing region or a thrombus capturing depressed region: the mesh basket 41 may be of a metal wire woven structure or a laser engraving structure. If the mesh basket is of the metal wire woven structure, the wire diameter range of the metal wire is 0.03-0.12 mm; if the mesh basket is of the laser engraving structure, the width range of stent struts of the laser engraving structure is 0.04-0.12 mm, and the thickness range of the stent struts is 0.03-0.10 mm. Therefore, the laser engraving structure has unique mechanical properties, such that the passing ability and safety thereof in the blood vessel are improved. The spatial form of the mesh basket 41 in the open state may be of different structures such as ellipsoidal shape, spindle shape, rugby shape, bulb shape, gourd shape, pear shape and dumb-bell shape, and the thrombectomy stent 4 may be formed by any combination of various mesh baskets 41 in different forms. Each thrombectomy stent formed by the mesh baskets 41 in different forms has the large mesh capturing region or the thrombus capturing depressed region which is capable of more effectively capturing the thrombus. The mesh area of the depressed region of an existing self-expanded stent is about 9.5 mm², and the mesh area of the large mesh capturing region or the thrombus capturing depressed region of the thrombectomy stent of the adjustable thrombectomy device may be adjusted from 5 mm² to 19.5 mm², preferably from 10 mm² to 18 mm².

The mesh basket 41 woven by the metal wire may be woven by a single strand of the metal wire or may be woven by 2-5 strands of the wound metal wires: the metal wire may be made from a material with a better developing property such as a nickel-titanium alloy, a platinum core nickel-titanium composite filament and a platinum wire, and has a shape memory function; the mesh basket 41 may be compositely woven by the above metal wires and may also be woven by the above single material.

The adjustable range of the length of the thrombectomy stent 4 is 10-60 mm; and the adjustable range of the diameter of the thrombectomy stent 4 is 1.0-6.0 mm.

The wire diameter range of the developing spring 51 at the distal end of the thrombectomy device is 0.02-0.06 mm, the length range of the developing spring 51 is 5-15 mm, and the diameter range of the developing spring 51 is 0.2-0.5 mm.

FIG. 7 is a schematic diagram of an open state of a thrombectomy stent of an adjustable thrombectomy device at a lesion part according to a third embodiment of the present disclosure. As shown in FIG. 7, the thrombectomy stent 4 is completely opened at the thrombus 8 at the lesion part within the blood vessel 7; the thrombectomy stent 4 includes two mesh baskets 41 same in size; the metal wire of the mesh basket 41 is formed by winding a plurality of strands of filaments; and the single mesh basket is in the ellipsoidal form.

FIG. 8 is a schematic diagram of an open state of a thrombectomy stent of an adjustable thrombectomy device at a lesion part according to a fourth embodiment of the present disclosure. As shown in FIG. 8, the thrombectomy stent 4 is completely opened at the thrombus 8 at the lesion part within the blood vessel 7; the thrombectomy stent 4 includes two mesh baskets 41 same in size; the metal wire of the mesh basket 41 is formed by winding a plurality of strands of filaments; and the single mesh basket is in the gourd shape.

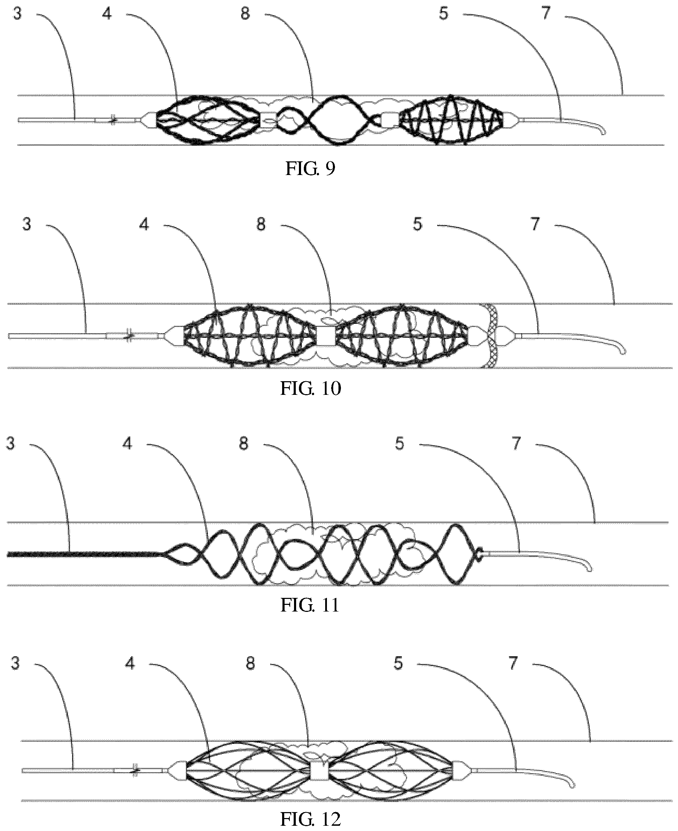

FIG. 9 is a schematic diagram of an open state of a thrombectomy stent of an adjustable thrombectomy device at a lesion part according to a fifth embodiment of the present disclosure. As shown in FIG. 9, the thrombectomy stent 4 is completely opened at the thrombus 8 at the lesion part within the blood vessel 7; the thrombectomy stent 4 includes four mesh baskets 41 different in size, wherein the two mesh baskets 4 in the middle are large and small, and the weaving meshes of the mesh baskets 41 are larger, which facilitates effective capture of large thrombi; the weaving meshes of the mesh baskets 41 at both ends are dense after being opened, and the mesh basket 41 at the most distal end may effectively prevent thrombi at the distal end from escaping in the thrombectomy process.

FIG. 10 is a schematic diagram of an open state of a thrombectomy stent of an adjustable thrombectomy device at a lesion part according to a sixth embodiment of the present disclosure. As shown in FIG. 10, the thrombectomy stent 4 is completely opened at the thrombus 8 at the lesion part within the blood vessel 7; the thrombectomy stent 4 includes two ellipsoidal mesh baskets and a disc-like mesh basket; the disc-like mesh basket is located at the most distal end of the thrombectomy stent 4; the outer diameter of the disc-like mesh basket is equal to the inner diameter of the blood vessel 7; the disc-like mesh basket opened may cling to the inner wall of the blood vessel 7 to effectively prevent thrombi at the distal end from escaping in the thrombectomy process. The outer diameter of the disc-like mesh basket is usually consistent with or slightly greater than the inner diameter of the blood vessel. In the open state, the outer diameter of the disc-like mesh basket is adaptive to the inner diameter of the blood vessel.

FIG. 11 is a schematic diagram of an open state of a thrombectomy stent of an adjustable thrombectomy device at a lesion part according to a seventh embodiment of the present disclosure. As shown in FIG. 11, the thrombectomy stent 4 is completely opened at the thrombus 8 at the lesion part within the blood vessel 7; the thrombectomy stent 4 includes seven mesh baskets 41 different in size, wherein two thrombus capturing depressed regions are formed in the middle section of the thrombectomy stent 4, thereby facilitating capture of the thrombus 8.

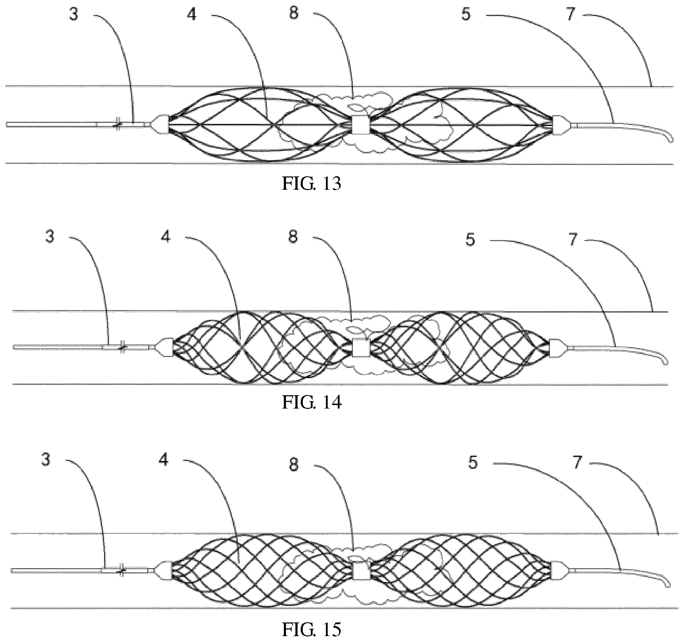

FIGs. 12-17 show structures in various different forms of the thrombectomy stent 4, and the structures all may be implemented either by the metal wire weaving structure or the laser engraving structure.

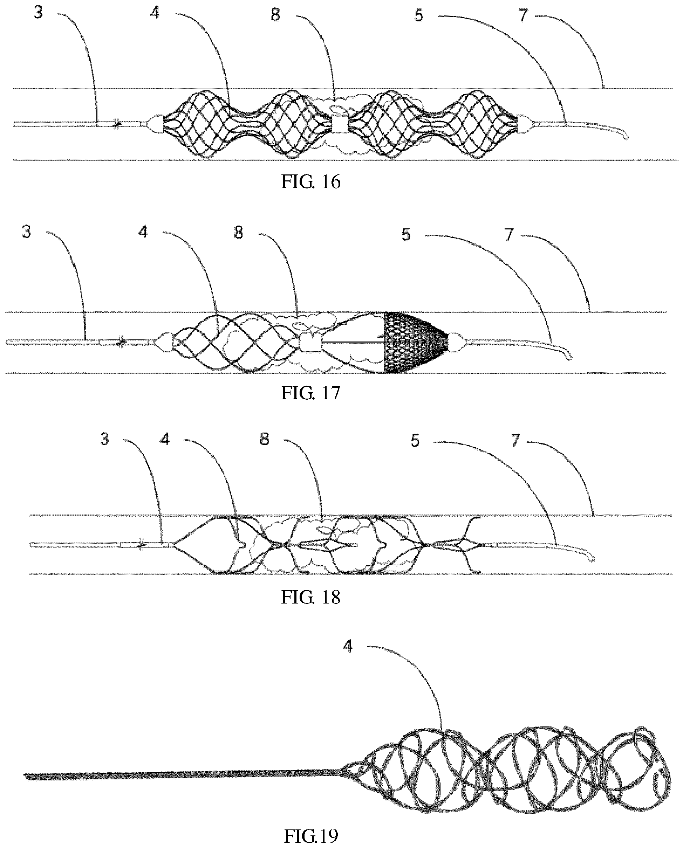

FIG. 18 is a schematic diagram of an open state of a thrombectomy stent of an adjustable thrombectomy device at a lesion part according to a ninth embodiment of the present disclosure. As shown in FIG. 18, the thrombectomy stent 4 is completely opened at the thrombus 8 at the lesion part within the blood vessel 7; the thrombectomy stent 4 is a novel stent with supporting rib structures inside and expands peripherally through the inside supporting rib structures; the principle of the structure is similar to the moving principle of an umbrella; the middle supporting rib is pulled by operating the adjusting filament 6 to shift backwards to expand the thrombectomy stent 4.

FIG. 19 is a schematic diagram of an open state of a thrombectomy stent of an adjustable thrombectomy device according to a tenth embodiment of the present disclosure at a lesion part; as shown in FIG. 19, in the completely open state, the thrombectomy stent 4 includes three ellipsoidal mesh baskets which are of an integrally woven structure, wherein there is no columnar connecting points between every two mesh baskets, and a functional depressed region beneficial to thrombus capture is formed between two adjacent mesh baskets.

The specific working process of the adjustable thrombectomy device provided by the embodiments of the present disclosure is carried out in the following way:

The adjustment knob 2 on the adjustable handle 1 is rotated or pushed and pulled to pull the adjusting filament 6, and the adjusting filament 6 tensions the thrombectomy stent 4, so the thrombectomy stent 4 expands to be opened at the thrombus 8 at the lesion part within the blood vessel 7 so as to capture the thrombus 8; after capture, the thrombectomy stent 4 and the captured thrombus together retreat to the middle conduit and are integrally withdrawn outside, so that the thrombus within the blood vessel is taken out.

The objects, technical solutions and beneficial effects of the present disclosure are further described in detail in the above specific implementations. It shall be understood that the above implementations are merely specific ones of the present disclosure and are not intended to limit the protection scope of the present disclosure. Any modification, equivalent substitution, improvement and the like made within the spirit and principle of the present disclosure shall be included in the protection scope of the present disclosure.

## Claims

1. An adjustable thrombectomy device, comprising:
an adjustable handle having a hollow structure and comprising an adjustment knob;
an adjusting filament having a proximal end connected to the adjustment knob, wherein the adjusting filament is configured to be adjusted by the adjustment knob to rotate and/or move axially;
a conveying conduit having a proximal end inserted into the hollow structure of the adjustable handle, wherein the conveying conduit is sleeved outside the adjusting filament;
a thrombectomy stent having a proximal end connected to a distal end of the adjusting filament, wherein the thrombectomy stent is configured to be pulled by the adjusting filament to expand or contract.

2. The adjustable thrombectomy device according to claim 1, wherein the adjustment knob has at least one of the following adjusting modes:
a rotating mode and a push-pull mode.

3. The adjustable thrombectomy device according to claim 1, further comprising:
a guide component connected to a distal end of the thrombectomy stent, wherein the guide component comprises a developing spring, and a ball head is arranged on the developing spring.

4. The adjustable thrombectomy device according to claim 1, wherein the thrombectomy stent comprises one mesh basket or a plurality of mesh baskets connected in series.

5. The adjustable thrombectomy device according to claim 4, wherein developable labels are arranged at proximal ends and/or distal ends of at least part of the mesh baskets.

6. The adjustable thrombectomy device according to claim 4, wherein the mesh basket is provided with a large mesh capturing region or a thrombus capturing depressed region.

7. The adjustable thrombectomy device according to claim 4, wherein the mesh basket is of a metal wire woven structure or a laser engraving structure.

8. The adjustable thrombectomy device according to claim 7, wherein a wire diameter range of metal wires of the metal wire woven structure is 0.03-0.12 mm.

9. The adjustable thrombectomy device according to claim 7, wherein a width range of stent struts of the laser engraving structure is 0.04-0.12 mm, and a thickness range of the stent struts is 0.03-0.10 mm.

10. The adjustable thrombectomy device according to claim 3, wherein a wire diameter range of the developing spring is 0.02-0.06 mm;
a length range of the developing spring is 5-15 mm; and
a diameter range of the developing spring is 0.2-0.5 mm.

11. The adjustable thrombectomy device according to claim 1, wherein an adjustable range of an overall length of the thrombectomy stent is 10-60 mm; and
a diameter adjustable range of the thrombectomy stent is 1.0-6.0 mm.

12. The adjustable thrombectomy device according to claim 4, wherein the mesh basket at the distal end of the thrombectomy stent is of a disc-like structure in an open state, and an outer diameter of the disc-like structure is adaptive to an inner diameter of the blood vessel.

13. The adjustable thrombectomy device according to claim 4, wherein the spatial form of the mesh basket of the thrombectomy stent in the open state is any one or more combinations of ellipsoidal shape, spindle shape, rugby shape, bulb shape, gourd shape, pear shape and dumb-bell shape.
